(19) Europäisches Patentamt
European Patent Office
Office européen des brevets

(11) Veröffentlichungsnummer: **0 138 020 B1**

# (12) EUROPÄISCHE PATENTSCHRIFT

(45) Veröffentlichungstag der Patentschrift: **03.06.92**

(51) Int. Cl.[5]: **A61K 31/135**, //(A61K31/135, 31:65,31:43,31:545,31:70)

(21) Anmeldenummer: **84110491.2**

(22) Anmeldetag: **04.09.84**

## (54) Antiadhäsive Prophylactica und Arzneimittel enthaltend ein sekretolytisch wirksames Benzylaminderivat.

(30) Priorität: **17.09.83 DE 3333632**
**23.11.83 DE 3342269**

(43) Veröffentlichungstag der Anmeldung:
**24.04.85 Patentblatt 85/17**

(45) Bekanntmachung des Hinweises auf die Patenterteilung:
**03.06.92 Patentblatt 92/23**

(84) Benannte Vertragsstaaten:
**BE CH DE FR GB IT LI LU NL SE**

(56) Entgegenhaltungen:
**DE-A- 2 416 142**
**FR-A- 2 516 795**
**US-A- 4 073 942**

**ARZNEIMITTEL FORSCHUNG, Band 31, Nr. 6, 1981, Seiten 974-976; J.C.M. WIEMEYER: "Influence of ambroxol on the bronchopulmonary level of antibiotics"**

(73) Patentinhaber: **Dr. Karl Thomae GmbH**
**Postfach 1755**
**W-7950 Biberach (Riss)(DE)**

(72) Erfinder: **Krüger, Christian, Prof. Dr.**
**Curtiusstrasse 4**
**W-2400 Lübeck(DE)**
Erfinder: **Erne, Angelika Maria Dr. Dipl.-Mikrobiol.**
**Beim Fohrhäldele 18**
**W-7950 Biberach 1(DE)**
Erfinder: **Werner, Rolf-Günther, Dr., Dipl.-Mikrobiol.**
**Beim Fohrhäldele 18**
**W-7950 Biberach 1(DE)**
Erfinder: **Goeth, Hanns, Dr.**
**Oberer Bühl 6**
**W-7950 Biberach 1(DE)**
Erfinder: **Ziegler, Harald, Dr.**
**Händelstrasse 10**
**W-7950 Biberach 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

In den US-A-3,336,308, 3,536,713 und 4,113,777 werden Benzylaminderivate und deren physiologisch verträgliche Säureadditionssalze mit anorganischen oder organischen Säuren beschrieben, welche neben einer hustenstillenden Wirkung insbesondere sekretolytische Eigenschaften aufweisen. Hierbei haben die beiden Verbindungen Bromhexin-hydrochlorid [N-Methyl-N-(2-amino-3,5-dibrom-benzyl)-cyclohexylamin-hydrochlorid] und Ambroxol-hydrochlorid [N-(2-Amino-3,5-dibrombenzyl)-trans-4-hydroxy-cyclohexylamin-hydrochlorid] Eingang in die Therapie als Sekretolytica gefunden.

Außerdem ist aus der FR-A-2,516,795 und aus Arzneimittelforschung 31, 974-976 (1981) bekannt, daß die vorstehend erwähnten Sekretolytika die Antibiotikaspiegel im Lungengewebe bzw. im Sputum erhöhen.

Überraschenderweise wurde nun gefunden, daß die alleinige Applikation eines Benzylamins der allgemeinen Formel

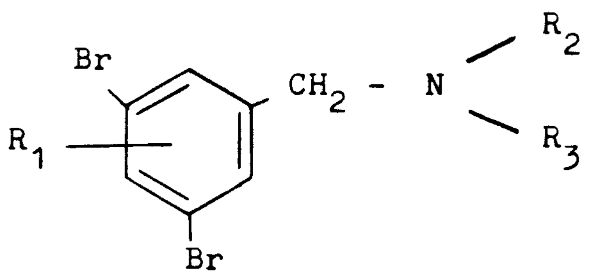

(I)

in der

$R_1$ eine Hydroxygruppe in 2- oder 4-Stellung oder eine Aminogruppe in 2-Stellung,

$R_2$ ein Wasserstoffatom oder eine Methylgruppe und

$R_3$ eine Cyclohexyl- oder Hydroxycyclohexylgruppe bedeuten, sowie deren physiologisch verträgliche Säureadditionssalze mit anorganischen oder organischen Säuren, die Besiedlung von Oberflächen mit Mikroorganismen behindert bzw. verhindert, insbesondere im Mund- und Rachenraum, in der Lunge, im Urogenitaltrakt, in den Brustdrüsen, auf der Haut und den Conjunktiven.

Bevorzugte Verbindungen der obigen allgemeinen Formel I sind diejenigen, in denen $R_2$ und $R_3$ zusammen mit dem dazwischen liegenden Stickstoffatom die N-Methyl-cyclohexylamino-, cis-3-Hydroxy-cyclohexylamino- oder trans-4-Hy-droxy-cyclohexylaminogruppe darstellen, insbesondere die Verbindungen

N-Methyl-N-(2-amino-3,5-dibrom-benzyl)-cyclohexylamin,

N-(2-Amino-3,5-dibrom-benzyl)-trans-4-hydroxy-cyclohexylamin,

N-(3,5-Dibrom-4-hydroxy-benzyl)-cis-3-hydroxy-cyclohexylamin

und

N-(3,5-Dibrom-2-hydroxy-benzyl)-trans-4-hydroxy-cyclohexylamin

sowie deren physiologisch verträgliche Säureadditionssalze mit anorganischen oder organischen Säuren.

Erfindungsgemäß wird somit bei der Applikation einer Verbindung der obigen allgemeinen Formel I die Gefahr einer Infektion durch Mikroben, insbesondere des Mund- und Rachenraumes, der Lunge, der Vagina und der Brustdrüsen durch Verringerung der Adhäsivität der Mikroben an den Zellwänden erschwert oder verhindert.

Beispielsweise wurde diese prophylaktische Wirkung der Verbindung

A = N-Methyl-N-(2-amino-3,5-dibrom-benzyl)-cyclohexylaminhydrochlorid und

B = N-(2-Amino-3,5-dibrom-benzyl)-trans-4-hydroxy-cyclohexylamin-hydrochlorid

wie folgt geprüft:

1. Direkte Wirkung im in vitro System

a. Der Titer der lebenden Zellen, z.B. von menschlichen Lungen-Epithelzellen wie ATCC, CCL5, L-132, - zu denen nachgewiesenerweise die Verbindungen der obigen allgemeinen Formel I eine sehr hohe Affinität aufweisen, die mit der zum intakten Organ vergleichbar ist - wird mittels Vitalfärbung mit Trypan blue und Auszählung in einer Fuchs-Rosenthal-Zählkammer bestimmt und durch Verdünnung mit Kulturmedium, z.B. Minimum Essential Medium plus 10 % fötales Kälberserum plus 0,1 % Kanamycin, auf 7 x $10^4$ Zellen/ml eingestellt. Je 3 ml dieser Suspension werden in Glasszintillationsfläschchen gefüllt, die durch eine übliche Behandlung für die Anheftung der Epithelzellen auf die Glasoberfläche vorbereitet wurden. Die Inkubation bis zur Ausbildung eines geschlossenen Monolayers erfolgt über 3

Tage bei 37°C und 3 % $CO_2$. Ein Monolayer entspricht 5 x $10^6$ Zellen pro Szintillationsfläschchen.

b. Die Bakterien, z.B. Klebsiella pneumoniae Weingarten 14, werden in zwei Passagen von je 24 Stunden bei 37° C in Brain Heart Infusion Broth (BHJ) bis zur logarithmischen Wachstumsphase gezüchtet. Während der 2. Passage erfolgt die radioaktive Markierung durch die Zugabe von 1,2-$^{14}$C-Azetat zum Nährmedium. Danach wird die Kultur zur Entfernung nichtinkorporierter Radioaktivität zweimal mit PBS (Phosphate buffered saline) gewaschen. Mit PBS pH 5,5 wird die Kultur im Photometer auf eine Transmission von 35 % eingestellt, was durch Ermittlung der Lebendkeimzahl einem Titer von 5 x $10^8$ Bakterien/ml entspricht.

c. Die Bakterien-Kultur wird abzentrifugiert, PBS abgenommen und durch ein gleiches Volumen Benzylamin-Lösung in PBS pH 5,5 ersetzt. Für den Kontrollwert wird statt der Benzylamin-Lösung PBS pH 5,5 verwendet.

Das Nährmedium der Zellen wird abgenommen, der Monolayer (= 5 x $10^6$ Zellen) wird zweimal mit PBS pH 5,5 gespült und mit 1 ml Bakterien-Benzylamin-Lösung (= 5 x $10^8$ Bakterien) überschichtet. Die Inkubation erfolgt 3 Stunden bei 37° C.

Danach wird die Bakterien-Benzylamin-Lösung abgenommen und der Monolayer zur Entfernung nicht-adhärenter Bakterien zweimal mit PBS pH 5,5 gespült.

Nach Zugabe von Instagel wird die Radioaktivität der gebundenen Bakterien im Szintillationszähler gemessen.

d. Ergebnis:

Mit der Verbindung A (50 |g/ml) wurden im Vergleich zur Kontrolle 15 % weniger Keime auf den Epithelzellen nachgewiesen, mit der Verbindung B (50 |g/ml) 20 %.

2. <u>Indirekte Wirkung im ex vivo System</u>

a. Die Kultur der Epithelzellen erfolgt wie oben beschrieben.

b. Die Kultur der Bakterien erfolgt wie oben beschrieben.

c. Männliche SPF-Ratten (Chbb:THOM) werden über 5 Tage p.o. mit 25 mg Benzylamin pro kg Körpergewicht bzw. mit Tylose (Kontrolle) behandelt. Durch Bronchoalveolar-Lavage mit isotoner Harnstofflösung wird das Lungenwaschwasser gewonnen und gepoolt. 100 ml davon werden mittels präparativer Isoelektrofokussierung in 30 proteinhaltige Gelfraktionen aufgetrennt. Nach Bestimmung des isoelektrischen Punktes der einzelnen Fraktionen mit einer Oberflächen-Elektrode werden die Proteine mit 3 ml PBS pH 5,5 pro Fraktion vom Gel eluiert. Die 30 Fraktionen werden auf pH 5,5 eingestellt.

d. Die Bakterienkultur wird abzentrifugiert, PBS abgenommen und durch ein gleiches Volumen Lungenwaschwasserfraktion ersetzt. Für den Kontrollwert wird statt der Lungenwaschwasserfraktionen PBS pH 5,5 und isotone Harnstofflösung verwendet. Nach einer Inkubation von 2 Stunden bei 37° C wird die Bakterienkultur erneut abzentrifugiert, Lungenwaschwasserfraktionen bzw. PBS bzw. Harnstoff abgenommen und durch ein gleiches Volumen PBS pH 5,5 ersetzt.

Das Nährmedium der Zellen wird abgenommen, der Monolayer (= 5 x $10^6$ Zellen) wird zweimal mit PBS pH 5,5 gespült und mit 1 ml Bakteriensuspension (= 5 x $10^8$ Bakterien) überschichtet. Die Inkubation erfolgt 2 Stunden bei 37° C Danach wird die Bakteriensuspension abgenommen und der Monolayer zur Entfernung nicht-adhärenter Bakterien zweimal mit PBS pH 5,5 gespült. Nach Zugabe von Instagel wird die Radioaktivität der gebundenen Bakterien im Szintillationszähler gemessen.

e. Ergebnis:

In wiederholten Versuchen mit der Verbindung B wurden bei 8 Fraktionen mit reproduzierbaren isoelektrischen Punkten adhäsionssteigernde Mediator-Proteine nachgewiesen. Bei behandelten Tieren wurde diese Mediator-bedingte erhöhte Adhäsivität im Vergleich zu den Kontrolltieren signifikant verringert. Die Ergebnisse sind in der nachfolgenden Tabelle zusammengefaßt.

| Isoelektrischer Punkt der Lungenwaschwasserfraktion | % Hemmung der Adhäsivität |
|---|---|
| 4,0 | 73 |
| 4,4 | 65 |
| 4,9 | 65 |
| 5,2 | 61 |
| 5,8 | 39 |
| 6,7 | 67 |
| 7,6 | 79 |
| 8,6 | 72 |

3. Wirkung im in vivo System

a. Die Kultur der Bakterien erfolgt wie oben beschrieben, allerdings ohne radioaktive Markierung.

b. Weibliche Albino-Wistar Ratten oder weibliche SPF-Mäuse (Chbb:NMRI) werden i.p., i.v., i.m., p.o., vorzugsweise i.m. 8-10 Stunden vor, 15 Minuten vor und 4-5 Stunden nach der Infektion mit 2-200 mg, vorzugsweise 20-40 mg Benzylamin pro kg Körpergewicht bzw. Tylose (Kontrolle) behandelt.

Die Bakteriensuspension (5 x $10^{8}$-$10^{-9}$ Bakterien/ml physiologischer Kochsalzlösung) wird in einer Vernebelungskammer mit 0,35 ml/ Minute über 10 Minuten vernebelt.

Alternativ werden $10^{6}$ Keime in 0,05 ml physiologischer Kochsalzlösung i.n. in den Nasengang unter einer Inhalationsnarkose, z.B. mit Chloroform, appliziert.

Jeweils ein Kollektiv von Tieren wird 1-10, vorzugsweise 10 Stunden nach der Infektion seziert, die Lungen steril entnommen und die Keime im Lungenhomogenat quantitativ durch Anzüchtung auf McConkey-Agar ermittelt. Die Bakterienkonzentration wird in Keimzahl/g Lunge bestimmt.

c. Ergebnis:

| Substanz | % Keimzahl/g Lunge |
|---|---|
| Tylose | 100 |
| Bromhexin-hydrochlorid | 49 |
| Ambroxol-hydrochlorid | 31 |

Erfindungsgemäß verhindern die Verbindungen der obigen allgemeinen Formel I beispielweise die Besiedlung durch Bakterien wie Actinomyces, Bacteroides, Bordetella pertussis, Corynebacterium diphteriae, Escherichia coli, Haemophilus influenzae, Klebsiella, Mycobacterium tuberculosis, Mycoplasma pneumoniae, Nocardia, Peptokokken, Peptostreptokokken, Pneumokokken, Proteus mirabilis, Pseudomonas aeruginosa, Serratia, Staphylokokken, Streptokokken und Pilze wie Aspergillus und Candida.

Die Verbindungen der obigen allgemeinen Formel I und deren physiologisch verträgliche Säureadditionssalze sind untoxisch und somit gut verträglich. So weist beispielsweise an der Maus

- N-Methyl-N-(2-amino-3,5-dibrom-benzyl)-cyclohexylaminhydrochlorid eine $LD_{50}$ von >10 g/kg p.o.,
- N-(2-Amino-3,5-dibrom-benzyl)-trans-4-hydroxy-cyclohexylaminhydrochlorid eine von 2,72 g/kg p.o. und
- die übrigen Verbindungen eine von >1 g/kg p.o. auf.

Aufgrund der erfindungsgemäß aufgefundenen Hemmwirkung auf die Adhäsivität von Mikroorganismen eignen sich die Verbindungen der obigen allgemeinen Formel I zur Prophylaxe von Infektionen wie solchen des Respirationstraktes, des Urogenitaltraktes, der Brustdrüsen, der Haut und der Conjunktiven, insbesondere auch zur Hygiene des Mund- und Rachenraumes.

Zur Erzielung der erfindungsgemäßen Wirkung lassen sich die Verbindungen der allgemeinen Formel I sowie deren physiologisch verträglichen Säureadditionssalze mit anorganischen oder organischen Säuren in die üblichen für den betreffenden Verwendungszweck geeigneten Zubereitungsformen wie Tabletten, Dragées, Kapseln, Pulver, Sprays, Pasten, Cremes, Salben, Suspensionen oder Lösungen und Wundpflaster einarbeiten.

Peroral werden hierbei die Verbindungen der allgemeinen Formel I in einer Dosis von 1 bis 200 mg, vorzugsweise jedoch von 8 bis 150 mg, gegebenenfalls mehrmals, beispielsweise 1 bis 4 x täglich, und äußerlich in einer Konzentration von 0,01 bis 20 mg/ml bzw. 0,01 bis 20 mg/g angewendet.

Die nachfolgenden Beispiele sollen die Erfindung näher erläutern, wobei in jedem Beispiel selbstverständlich der Wirkstoff durch einen anderen der allgemeinen Formel I ausgetauscht werden kann.

Beispiel 1

| Tabletten zu 8 ma Bromhexin-hydrochlorid | |
|---|---|
| 1 Tablette enthält: | |
| Bromhexin-hydrochlorid | 8,0 mg |
| Milchzucker | 50,0 mg |
| Kartoffelstärke | 49,0 mg |
| Polyvinylpyrrolidon | 2,0 mg |
| Magnesiumstearat | 1,0 mg |
| | 110,0 mg |

Herstellungsverfahren:

Wirkstoff mit Milchzucker und Kartoffelstärke mischen, mit einer wäßrigen Polyvinylpyrrolidon-Lösung gleichmäßig befeuchten, die Masse durch ein 1,5 mm-Sieb geben, das Granulat im Umlufttrockenschrank bei ca. 40 °C trocknen und anschließend durch ein Sieb mit 1,0 mm-Maschenweite passieren. Nach Zumischung des Schmiermittels werden die Tabletten gepreßt.

Stempel: 7 mm, biplan mit beidseitiger Facette, Teilkerbe auf einer Seite
Tablettengewicht: 110 mg.

Beispiel 2

| Tabletten zu 60 ma Ambroxol-hydrochlorid | |
|---|---|
| 1 Tablette enthält: | |
| Ambroxol-hydrochlorid | 60,0 mg |
| Milchzucker | 50,0 mg |
| Kartoffelstärke | 47,0 mg |
| Polyvinylpyrrolidon | 2,0 mg |
| Magnesiumstearat | 1,0 mg |
| | 160,0 mg |

Herstellungsverfahren:

analog Beispiel 1.

Stempel: 8 mm, biplan mit beidseitiger Facette, Teilkerbe auf einer Seite
Tablettengewicht: 160 mg

Beispiel 3

| Ampullen zu 8 mg Bromhexin-hydrochlorid | |
|---|---|
| 1 Ampulle enthält: | |
| Bromhexin-hydrochlorid | 8,0 mg |
| Weinsäure | 4,0 mg |
| Traubenzucker | 190,0 mg |
| Wasser für Injektionszwecke ad | 4,0 ml |

Herstellungsverfahren: (100 1)

In 90 1 ca. 80 °C warmem Wasser unter $N_2$-Begasung und Rühren die entsprechende Menge Weinsäure und Bromhexin-hydrochlorid lösen. Nach Abkühlen auf Raumtemperatur unter $N_2$-Begasung dann Traubenzucker lösen und auf 100 1 auffüllen. Nach Sterilfiltration und Abfüllen in 2-ml-Ampullen 20 Minuten bei 120 °C sterilisieren.

Beispiel 4

| Ampullen zu 15 mg Ambroxol-hydrochlorid | | |
|---|---|---|
| 1 Ampulle enthält: | | |
| Ambroxol-hydrochlorid | (1) | 15,00 mg |
| Citronensäure | (2) | 1,83 mg |
| Natrium-monohydrogenphosphat | (3) | 7,24 mg |
| Natriumchlorid | (4) | 14,40 mg |
| Wasser für Injektionszwecke ad | | 2,00 ml |

Herstellungsverfahren:

In erwärmtem Wasser werden nacheinander 2, 1, 3 und 4 gelöst. Nach Abkühlen auf Raumtemperatur unter $N_2$-Begasung auf 100 1 auffüllen. Nach Sterilfiltration und Abfüllen in 2-ml-Ampullen 20 Minuten bei 120 °C sterilisieren.

Beispiel 5

| Saft mit 8 mg Bromhexin-hydrochlorid pro 5 ml | |
|---|---|
| 100 ml enthalten: | |
| Bromhexin-hydrochlorid | 0,16 g |
| Zitronensäure | 0,50 g |
| Natriumhydroxid | 0,04 g |
| Natriumbenzoat | 0,20 g |
| Glycerin | 20,00 g |
| Sorbitlösung 70%ig | 70,00 g |
| Äthanol | 5,00 g |
| Naphtholrot S | 0,0003 g |
| Pflaumenaroma | 0,20 g |
| Dest. Wasser ad | 100,00 ml |

Herstellung:

Dest. Wasser wird auf 80 °C erhitzt und unter Rühren nacheinander Zitronensäure, Natriumhydroxid, Bromhexin-hydrochlorid, Glycerin und Sorbitlösung zugegeben und gelöst. Nach Abkühlen auf Raumtemperatur wird das Natriumbenzoat und Naphtholrot S zugegeben und gelöst sowie die Lösung des Aromas in der angegebenen Menge Alkohol zugemischt.

Beispiel 6

| Suppositorien zu 8 mg Bromhexin-hydrochlorid für Kinder | |
|---|---|
| 1 Zäpfchen enthält: | |
| Bromhexin-hydrochlorid | 0,0080 g |
| Polyäthylenglykol 1500 | 0,3238 g |
| Polyäthylenglykol 6000 | 0,2726 g |
| Polysorbat 60 (Tween 60) | 0,4850 g |
| Dinatriumhydrogenphosphat-Dihydrat | 0,0106 g |
| | 1,10 g |

Herstellungsverfahren:

Bromhexin-hydrochlorid und Dinatriumhydrogenphosphat werden getrennt gemahlen. Polysorbat 60 wird erwärmt und darin nacheinander Dinatriumhydrogenphosphat-Dihydrat und Bromhexin-hydrochlorid suspendiert.

Die Wirkstoffsuspension wird in die auf gleiche Temperatur gebrachte Schmelze der beiden Polyäthylenglykole eingetragen und diese Endmischung homogenisiert bevor sie daraufhin in vorgekühlte Zäpfchenformen ausgegossen wird.

Zäpfchengewicht: 1,1 g

Beispiel 7

| Ambroxol-hydrochlorid-Lösung zu 7,5 mg/ml zur Inhalation | |
|---|---|
| 100 ml enthalten: | |
| Ambroxol-hydrochlorid | 0,75 g |
| Glycerin 85 % | 5,00 g |
| prim. Natriumphosphat | 0,60 g |
| sek. Natriumphosphat | 0,055 g |
| Benzoesäure | 0,10 g |
| Dest. Wasser ad | 100,00 ml |

Herstellungsverfahren:

In dest. Wasser von 50-55 °C werden nacheinander Glycerin 85 %, prim. und sek. Natriumphosphat sowie Benzoesäure klar gelöst. Nach Abkühlen auf 25 °C wird Ambroxol-hydrochlorid gelöst und anschließend filtriert.

Beispiel 8

| Ambroxol-hydrochlorid-Lösung zu 10 mg/ml als Wundspüler | |
|---|---|
| 100 ml enthalten: | |
| Ambroxol-hydrochlorid<br>Sorbitlösung 70 %<br>Zitronensäure . $H_2O$<br>Natriumhydroxid<br>Natriumchlorid<br>Benzalkoniumchlorid<br>Dest. Wasser ad | 1,00 g<br>2,00 g<br>0,40 g<br>0,23 g<br>0,10 g<br>0,025 g<br>100,0 ml |

Herstellungsverfahren:

In dest. Wasser werden Sorbitlösung 70 %, Zitronensäure, Natriumchlorid und Benzalkoniumchlorid unter Rühren klar gelöst. Nach völliger Klarheit wird Natriumhydroxid zugefügt und gelöst. pH-Wert: 6,2; isotonisch.

Beispiel 9

Lutschtabletten mit 30 mg Ambroxol-hydrochlorid

| Zusammensetzung: | |
|---|---|
| 1 Tablette enthält: | |
| Ambroxol-hydrochlorid<br>Sorbitol, direkttablettierbar<br>Magnesiumstearat | 30,0 mg<br>666,5 mg<br>3,5 mg<br>700,0 mg |

Herstellungsverfahren:

Der Wirkstoff wird mit der gleichen Menge an Sorbitol homogen gemischt, dann werden die restlichen Tablettenbestandteile zugegeben und wieder eine homogene Mischung hergestellt = preßfertige Mischung. Letztere wird auf einer geeigneten Tablettiermaschine zu Tabletten verpreßt.

Gewicht: 700 mg
Durchmesser: 13 mm, Wölbungsradius 16 mm
Aussehen: weiß, rund, bikonvex

Beispiel 10

| Lutschtabletten mit 8 mg Bromhexin-hydrochlorid | |
|---|---|
| 1 Tablette enthält: | |
| Bromhexin-hydrochlorid<br>Sorbitol, direkttablettierbar<br>Magnesiumstearat | 8,0 mg<br>688,5 mg<br>3,5 mg<br>700,0 mg |

Herstellungsverfahren:

analog Beispiel 9.

Beispiel 11

| Tropfen mit 7,5 mg Ambroxol-hydrochlorid pro 1 ml | |
|---|---|
| 100 ml Tropflösung enthalten: | |
| Ambroxol-hydrochlorid | 0,75 g |
| Glycerin 85 % | 5,00 g |
| Citronensäure-Monohydrat | 0,20 g |
| Natrium-monohydrogenphosphat-dihydrat | 0,315 g |
| Natriumchlorid | 0,59 g |
| Saccharin-Natrium | 0,025 g |
| Aroma | 0,05 g |
| Benzoesäure | 0,20 g |
| Dest. Wasser ad | 100,00 ml |

Herstellungsverfahren:

Analog Beispiel 4.

## Patentansprüche

**1.** Verwendung eines Benzylamins der allgemeinen Formel

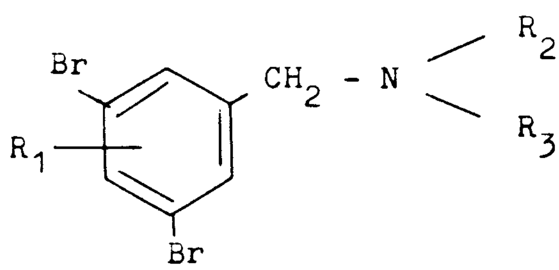

(I)

in der
$R_1$ eine Hydroxygruppe in 2- oder 4-Stellung oder eine Aminogruppe in 2-Stellung,
$R_2$ ein Wasserstoffatom oder eine Methylgruppe und
$R_3$ eine Cyclohexyl- oder Hydroxycyclohexylgruppe bedeuten, oder deren physiologisch verträglichen Säureadditionssalze mit anorganischen oder organischen Säuren als alleinigen Wirkstoff zur Herstellung eines Arzneimittels zur Prophylaxe von mikrobiellen Infektionen.

**2.** Verwendung gemäß Anspruch 1, dadurch gekennzeichnet, daß man die Verbindung der allgemeinen Formel I, in der $R_2$ und $R_3$ zusammen mit dem dazwischen liegenden Stickstoffatom die N-Methyl-cyclohexylamino- oder cis-3-Hydroxy-cyclohexylaminogruppe darstellt, oder deren physiologisch verträglichen Säureadditionssalze verwendet.

**3.** Verwendung gemäß Anspruch 1, dadurch gekennzeichnet, daß man die Verbindung
N-Methyl-N-(2-amino-3,5-dibrom-benzyl)-cyclohexylamin,
N-(2-Amino-3,5-dibrom-benzyl)-trans-4-hydroxy-cyclohexylamin,
N-(3,5-Dibrom-4-hydroxy-benzyl)-cis-3-hydroxy-cyclohexylamin
oder
N-(3,5-Dibrom-2-hydroxy-benzyl)-trans-4-hydroxy-cyclohexylamin oder deren physiologisch verträglichen Säureadditionssalze verwendet.

4. Verwendung gemäß Anspruch 1, dadurch gekennzeichnet, daß man die Verbindung N-Methyl-N-(2-amino-3,5-dibrom-benzyl)-cyclohexylamin oder N-(2-Amino-3,5-dibrom-benzyl)-trans-4-hydroxy-cyclohexylamin oder deren physiologisch verträglichen Säureadditionssalze verwendet.

5. Verwendung gemäß den Ansprüchen 1 bis 4, dadurch gekennzeichnet, daß die Infektion eine Infektion des Respirationstraktes, des Urogenitaltraktes, der Brustdrüsen, der Haut, der Konjunktiven oder des Mund- und Rachenraumes ist.

## Claims

1. Use of a benzylamine of general formula

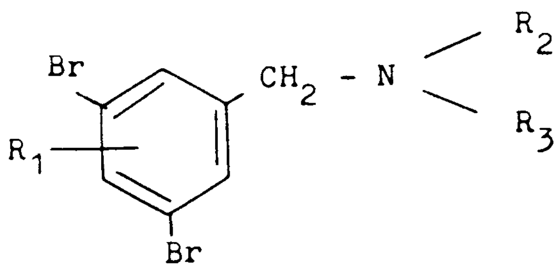

(I)

wherein
$R_1$ represents a hydroxy group in the 2- or 4-position or an amino group in the 2-position,
$R_2$ represents a hydrogen atom or a methyl group and
$R_3$ represents a cyclohexyl or hydroxycyclohexyl group, or the physiologically acceptable acid addition salts thereof with organic or inorganic acids as the sole active substance for preparing a pharmaceutical composition for the prevention of microbial infections.

2. Use according to claim 1, characterised in that the compound of general formula I used is one wherein $R_2$ and $R_3$ together with the nitrogen atom between them represent an N-methyl-cyclohexylamino or cis-3-hydroxy-cyclohexylamino group, or a physiologically acceptable acid addition salt thereof.

3. Use according to claim 1, characterised in that the compound N-methyl-N-(2-amino-3,5-dibromo-benzyl)-cyclohexylamine, N-(2-amino-3,5-dibromobenzyl)-trans-4-hydroxy-cyclohexylamine, N-(3,5-dibromo-4-hydroxybenzyl)-cis-3-hydroxy-cyclohexylamine or N-(3,5-dibromo-2-hydroxybenzyl)-trans-4-hydroxy-cyclohexylamine or a physiologically acceptable acid addition salt thereof is used.

4. Use according to claim 1, characterised in that the compound N-methyl-N-(2-amino-3,5-dibromobenzyl)-cyclohexylamine or N-(2-amino-3,5-dibromobenzyl)-trans-4-hydroxy-cyclohexylamine or a physiologically acceptable acid addition salt thereof is used.

5. Use according to claims 1 to 4, characterised in that the infection is an infection of the respiratory tract, the urogenital tract, the mammary glands, the skin, the conjunctiva or the oral or pharyngeal cavity.

## Revendications

1. Utilisation d'une benzylamine de formule générale

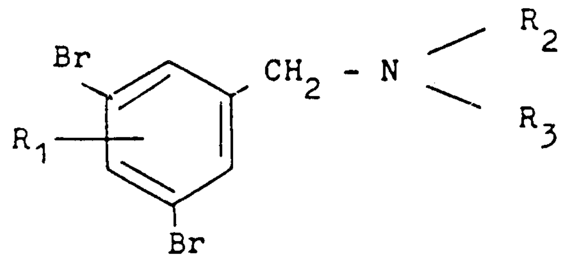

(I)

dans laquelle
$R_1$ représente un groupe hydroxyle en position 2 ou 4 ou un groupe amino en position 2,
$R_2$ représente un atome d'hydrogène ou un groupe méthyle et $R_3$ représente un groupe cyclohexyle ou hydroxycyclohexyle,
ou de ses sels d'addition d'acide compatibles du point de vue physiologique avec des acides inorganiques ou organiques comme principe actif unique pour la préparation d'un médicament pour la prophylaxie des infections microbiennes.

**2.** Utilisation selon la revendication 1, caractérisée en ce que l'on utilise le composé de formule générale I dans laquelle $R_2$ et $R_3$ représentent avec l'atome d'azote situé entre eux le groupe N-méthyl-cyclohexylamino ou cis-3-hydroxy-cyclohexylamino, ou ses sels d'addition d'acide compatibles du point de vue physiologique.

**3.** Utilisation selon la revendication 1, caractérisée en ce que l'on utilise le composé
N-méthyl-N-(2-amino-3,5-dibromobenzyl)-cyclohexylamine,
N-(2-amino-3,5-dibromobenzyl)-trans-4-hydroxy-cyclohexylamine,
N-(3,5-dibromo-4-hydroxy-benzyl)-cis-3-hydroxy-cyclohexylamine
ou
N-(3,5-dibromo-2-hydroxy-benzyl)-trans-4-hydroxy-cyclohexylamine
ou ses sels d'addition d'acide compatibles du point de vue physiologique.

**4.** Utilisation selon la revendication 1, caractérisée en ce que l'on utilise le composé
N'méthyl-N-(2-amino-3,5-dibromobenzyl)-cyclohexylamine ou
N-(2-amino-3,5-dibromobenzyl)-trans-4-hydroxy-cyclohexylamine
ou ses sels d'addition d'acide compatibles du point de vue physiologique.

**5.** Utilisation selon les revendications 1 à 4, caractérisée en ce que l'infection est une infection des voies respiratoires, des voies urogénitales, des glandes mammaires, de la peau, des conjonctives ou de la région de la bouche et de la gorge.